# EUROPEAN PATENT APPLICATION

(11) **EP 3 199 090 A1**
(43) Date of publication of application: **02.08.2017**
(21) Application number: 16832554.6
(22) Date of filing: 23.03.2016
(51) Int. Cl.: A61B 1/00

(54) **ENDOSCOPE FLEXIBLE TUBE**

(30) Priority: 04.08.2015 JP 2015154274
(71) Applicant: Olympus Corporation, Tokyo 192-8507 (JP)
(72) Inventor: YANO, Tetsuya, Tokyo 192-8507 (JP); MACHIDA, Yasushi, Tokyo 192-8507 (JP)
(74) Representative: Gunzelmann, Rainer
(86) International application number: PCT/JP2016/059131
(87) International publication number: WO 2017/022274

(57) **Abstract**

The present invention is to provide an endoscope flexible tube that makes it possible to secure larger internal space while maintaining assemblability. For the object, the endoscope flexible tube according to the present invention includes: a spiral tube 11 configured such that a belt-like plate is spirally wound; an inner cylindrical member 14 disposed inside an end part 21 of the spiral tube; and an outer cylindrical member 15 that is disposed outside the end part of the spiral tube and holds the end part of the spiral tube between the outer cylindrical member and the inner cylindrical member. The inner cylindrical member is disposed inside the end part of the spiral tube to make a diameter D1A of the end part of the spiral tube larger than a diameter D1 of a part that is different from the end part of the spiral tube.

## Description

### Technical Field

The invention relates to a configuration of an endoscope flexible tube that is inserted into an endoscope insertion section in order to realize flexibility of the endoscope insertion section.

### Background Art

An endoscope having an elongated tubular insertion section has been conventionally widely used, for example, in a medical field and an industrial field. Among the endoscopes, a medical endoscope used in the medical field is configured in such a manner that a flexible insertion section can be inserted into a body cavity of a subject such as a living body to allow for observation of organs or the like, or various kinds of treatment can be performed on the organs or the like with use of treatment instruments inserted into a treatment instrument insertion channel provided in the endoscope, as necessary. In addition, an industrial endoscope used in the industrial field is configured in such a manner that a flexible insertion section can be inserted into an object, for example, an apparatus such as a jet engine or an inside of equipment such as factory piping to allow for observation, inspection of a state and the like inside the object, for example, damage and corrosion.

The insertion section of the endoscope of the kind includes a distal end rigid portion, a bending portion, and a flexible tube that are connected in order from distal end side of the insertion section, and proximal end side of the flexible tube is coupled and fixed to a distal end of an operation section. Here, the flexible tube typically has a three-layer structure of a metal flex layer (a spiral tube), a metal braid layer (a mesh tube), and an elastomer outer resin layer (an outer skin). In addition, an inner pipe sleeve is disposed inside the flex layer of the flexible tube and an outer pipe sleeve is disposed on outer diameter side of the flex layer, at a coupling fixing portion between the proximal end side of the flexible tube and the distal end side of the operation section. This causes the flexible tube to be held between the inner pipe sleeve and the outer pipe sleeve. The inner pipe sleeve has an outer diameter smaller than an inner diameter of the flex layer. In the state, the outer pipe sleeve is swaged to fix the flexible tube. The endoscope flexible tube having such a configuration has been disclosed by, for example, Japanese Patent Application Laid-Open Publication No. 2009-153714, and has been widely practiced.

In contrast, an endoscope flexible tube disclosed by, for example, Japanese Patent Application Laid-Open Publication No. 2009-261645 has a configuration in which the flexible tube and the bending portion are arranged in contact with each other and a contact portion is welded to couple the flexible tube to the bending portion.

In the configuration disclosed by, for example, Japanese Patent Application Laid-Open Publication No. 2009-153714, however, it is necessary to make the outer diameter of the inner pipe sleeve smaller than the inner diameter of the flex layer. This decreases an inner region of the inner pipe sleeve (on the operation section side), which disadvantageously limits a storage quantity of built-in components to be disposed in the inner region. In addition, a large deformation amount of the outer pipe sleeve is necessary in order to obtain sufficient fixation force because the diameter of the inner pipe sleeve is made small. This increases a swaging amount of the outer pipe sleeve, which results in overload with respect to the apparatus. Further, occurrence of buckling of the outer pipe sleeve may occur, which disadvantageously makes stable processing difficult.

In contrast, means disclosed by, for example, Japanese Patent Application Laid-Open Publication No. 2009-261645 performs welding as bonding means of the contact portion between the flexible tube and the bending portion. This is disadvantageously inferior to the swaging work in workability.

The present invention is made in consideration of the above-described situation, and an object of the present invention is to provide an endoscope flexible tube that makes it possible to secure larger internal space while maintaining efficient and simple assemblability by simple means such as swaging fixation.

### Disclosure of Invention

### Means for Solving the Problem

To realize the above-described object, an endoscope flexible tube according to an aspect of the present invention includes: a spiral tube configured such that a belt-like plate is spirally wound; an inner cylindrical member disposed inside an end part of the spiral tube; and an outer cylindrical member that is disposed outside the end part of the spiral tube and holds the end part of the spiral tube between the outer cylindrical member and the inner cylindrical member. The inner cylindrical member is disposed inside the end part of the spiral tube to make a diameter of the end part of the spiral tube larger than a diameter of a part that is different from the end part of the spiral tube.

The present invention makes it possible to provide an endoscope flexible tube that makes it possible to secure larger internal space while maintaining efficient and simple assemblability through simple means such as swaging fixation.

### Brief Description of the Drawings

Fig. 1 is a perspective view illustrating an entire configuration of an endoscope to which an endoscope flexible tube according to a first embodiment of the present invention is applied;
Fig. 2 is a main-part exploded cross-sectional diagram illustrating a portion of components (a three-layered tube and an inner pipe sleeve) on proximal end side of an endoscope flexible tube according to a second embodiment of the present invention, in an exploded manner;
Fig. 3 is a cross-sectional diagram illustrating a state in which the three-layered tube and the inner pipe sleeve that are in an exploded state in Fig. 2 are assembled;
Fig. 4 is a cross-sectional diagram illustrating a state in which an end part of the three-layered tube and an end part of the inner pipe sleeve are inserted into an outer pipe sleeve, in the endoscope flexible tube according to the first embodiment of the present invention;
Fig. 5 is a cross-sectional diagram illustrating a state in which the end part of the three-layered tube and the end part of the inner pipe sleeve have been assembled to the outer pipe sleeve (a state in which swaging work has been completed), in the endoscope flexible tube according to the first embodiment of the present invention;
Fig. 6 is a main-part exploded cross-sectional diagram illustrating a portion of components on the proximal end side of the endoscope flexible tube according to a second embodiment of the present invention, and illustrating the three-layered tube and the inner pipe sleeve in an exploded manner (corresponding to Fig. 2 of the first embodiment);
Fig. 7 is a cross-sectional diagram illustrating a state in which the three-layered tube and the inner pipe sleeve that are in an exploded state in Fig. 6 are assembled (corresponding to Fig. 3 of the first embodiment);
Fig. 8 is a cross-sectional diagram illustrating a state in which an end part of the three-layered tube and an end part of the inner pipe sleeve are inserted into an outer pipe sleeve, in the endoscope flexible tube according to the second embodiment of the present invention (corresponding to Fig. 4 of the first embodiment); and
Fig. 9 is a cross-sectional diagram illustrating a state in which the end part of the three-layered tube and the end part of the inner pipe sleeve have been assembled to the outer pipe sleeve (a state in which swaging work has been completed), in the endoscope flexible tube according to the second embodiment of the present invention (corresponding to Fig. 5 of the first embodiment).

### Best Mode for Carrying Out the Invention

The present invention is described below based on illustrated embodiments. The respective drawings used in the following description are schematic illustration, and dimension relationship, scale sizes, and the like of respective members may be varied in illustration for each component in order to illustrate the components with respective recognizable sizes in the drawings. Therefore, the present invention is not limited to illustrated forms, in terms of the number of components, shapes of the respective components, a size ratio of the components, relative positional relationship between the components, and the like that are illustrated in the respective drawings.

### [First Embodiment]

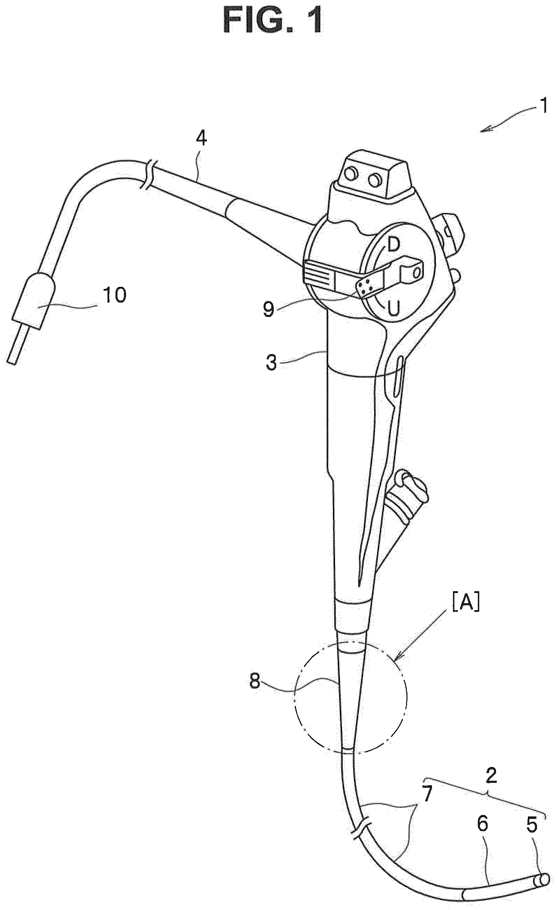

Fig. 1 is a perspective view illustrating an entire configuration of an endoscope to which an endoscope flexible tube according to a first embodiment of the present invention is applied.

Before detailed description of the endoscope flexible tube according to the first embodiment of the present invention, the entire configuration of the endoscope to which the endoscope flexible tube is applied is first described below with reference to Fig. 1.

An endoscope 1 to which the endoscope flexible tube (hereinafter, simply referred to as the flexible tube) according to the first embodiment of the present invention is applied mainly includes an insertion section 2, an operation section 3, and a universal cable 4, as illustrated in Fig. 1.

Although not illustrated, a light guide cable, an air/water feeding tube, various signal lines, and an internal structure such as a treatment instrument insertion channel are inserted into the insertion section 2, the operation section 3, and the universal cable 4.

The insertion section 2 is a component that is so formed in an elongated flexible tubular shape as a whole as to be insertable into a body cavity or the like. The insertion section 2 includes a distal end portion 5, a bending portion 6, and a flexible tube 7 in order from distal end side of the insertion section 2.

The distal end portion 5 includes, on a distal end surface, an observation window, an illumination window, a treatment instrument opening, a cleaning nozzle, etc. (not illustrated). In addition, an observation optical system, an image pickup device, etc. (not illustrated) are provided inside the distal end portion 5. The observation optical system and the image pickup device are connected to rear side of the observation window. Various kinds of signal lines and other lines are extended backward from the image pickup device. Further, a distal end of the light guide cable (not illustrated) is connected to rear side of the illumination window, inside the distal end portion 5. The light guide cable transmits, to the distal end portion 5, illumination light that is emitted from an unillustrated illumination apparatus. Likewise, a distal end of the air/water feeding tube (not illustrated) is connected to the cleaning nozzle, inside the distal end portion 5. The air/water feeding tube is extended from an unillustrated air/water feeding apparatus, and passes through the insertion section 2, the operation section 3, and the universal cable 4 and reaches the distal end portion 5. Furthermore, a distal end of the treatment instrument insertion channel is connected to rear side of the treatment instrument opening, inside the distal end portion 5.

The bending portion 6 is a component that makes a predetermined range of the distal end side of the insertion section 2 bendable. It is assumed that the bending portion 6 itself has a configuration similar to a bending portion of an endoscope that is in practical use generally and widely spread, and the description of the configuration is omitted.

The flexible tube 7 is a tubular member having flexibility as a whole. One end on distal end side of the flexible tube 7 is connected to a proximal end of the bending portion 6, and the other end on proximal end of the flexible tube 7 is connected to a distal end of the operation section 3. Note that the detailed configuration of the flexible tube 7 is described later.

The operation section 3 is a housing section that has an internal space. The proximal end of the insertion section 2 is connected to the operation section 3. A plurality of operation members and the like are provided on an outer surface of the operation section 3. The various kinds of signal lines, the light guide, an air/water feeding conduit, and the like that are extended from the insertion section 2 are inserted into the operation section 3, and an electric substrate, or the like on which an electronic circuit and other parts are mounted is disposed inside the operation section 3. The electronic circuit receives input signals respectively from the plurality of operation members. Examples of the plurality of operation members may include a bending lever 9 that is used to perform bending operation. Note that a connection site between the flexible tube 7 and the operation section 3 is covered with a bend preventing portion 8 and is thus protected from outside.

The universal cable 4 is a tubular cable that is extended from the operation section 3, and the various kinds of signal lines, the light guide, the various kinds of air/water feeding conduits, and the like are inserted into the universal cable 4. A connector portion 10 is connected to distal end side of the universal cable 4. The connector portion 10 is a connection member that is provided to be detachable from an unillustrated control unit and secures electrical and mechanical connection between the endoscope 1 and the control unit.

Note that it is assumed that the endoscope 1 has configurations substantially similar to configurations of the endoscope that is in practical use generally and widely spread in terms of more detailed configuration and other components, and the detailed description and illustration of the more detailed configuration and the other components are accordingly omitted.

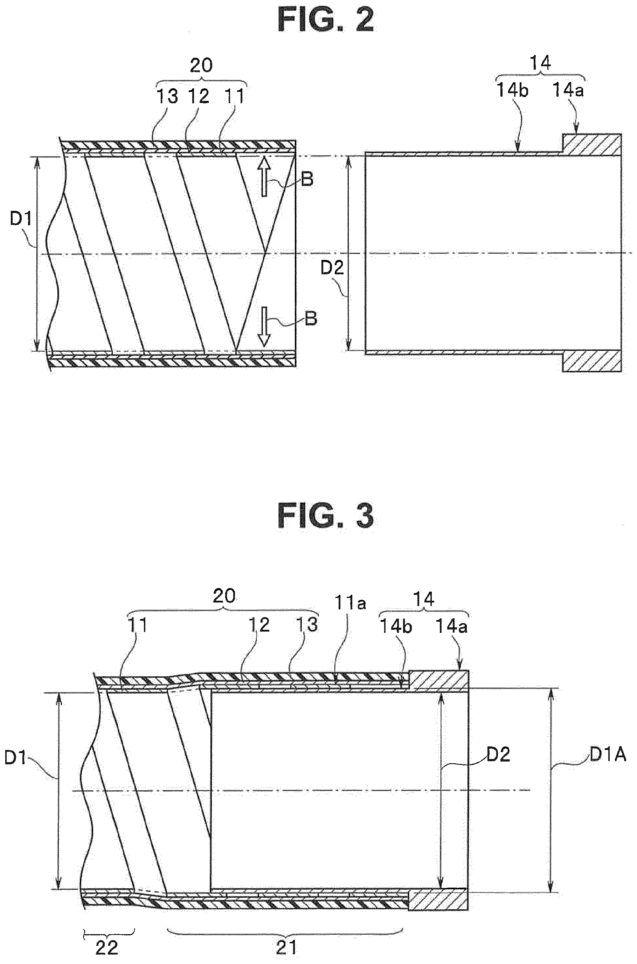

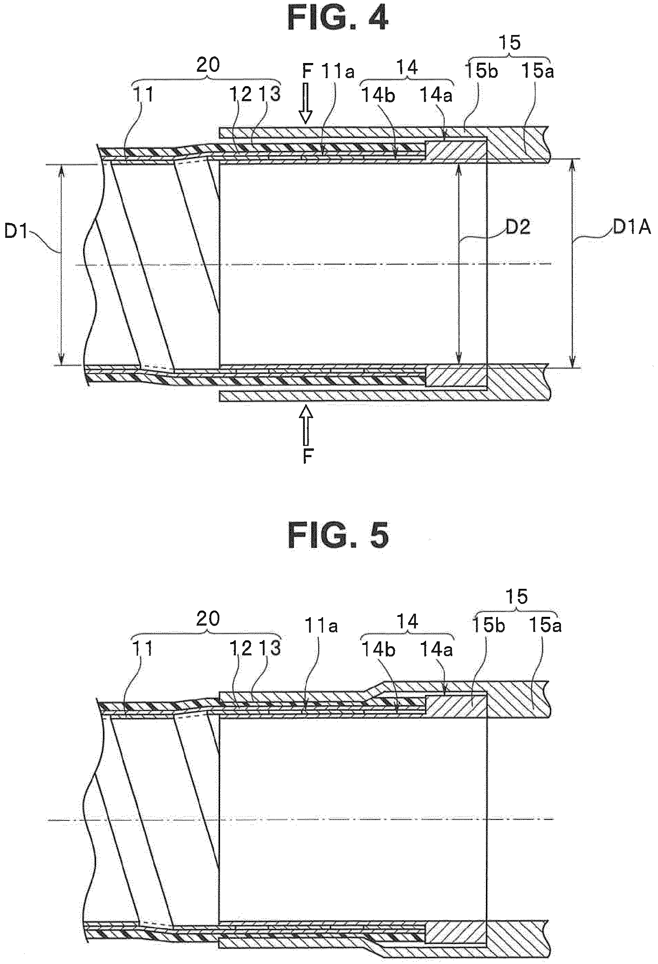

Next, the detailed configuration of the endoscope flexible tube 7 according to the present embodiment is described below with reference to Fig. 2 to Fig. 5. Fig. 2 to Fig. 5 are vertical cross-sectional diagrams each illustrating the configuration on the proximal end side of the endoscope flexible tube according to the present embodiment, in a simplified manner. Among the drawings, Fig. 2 is a diagram illustrating a portion of components on the proximal end side of the endoscope flexible tube according to the present embodiment, and is a main-part exploded cross-sectional diagram illustrating a three-layered tube and an inner pipe sleeve in an exploded manner. Fig. 3 is a diagram illustrating a state in which the three-layered tube and the inner pipe sleeve that are in an exploded state in Fig. 2 are assembled. Fig. 4 is a diagram illustrating a state in which an end part of the three-layered tube and an end part of the inner pipe sleeve are inserted into an outer pipe sleeve, in the endoscope flexible tube according to the present embodiment. Fig. 5 is a diagram illustrating a state in which the end part of the three-layered tube and the end part of the inner pipe sleeve have been assembled to the outer pipe sleeve (a state in which swaging work has been completed), in the endoscope flexible tube according to the present embodiment.

Note that Fig 2 to Fig. 5 are diagrams each illustrating an internal configuration of a site denoted by a reference sign [A] of Fig. 1, namely, a configuration near a coupling site between the proximal end of the endoscope flexible tube and the distal end of the operation section, in an enlarged manner.

As illustrated in Fig. 2 and Fig. 3, a main part of the flexible tube 7 has a three-layer structure that includes a spiral tube 11 serving as a flex layer, a mesh tube 12 serving as a braid layer, and an outer skin 13 serving as an outer resin layer.

The spiral tube 11 serving as the flex layer is a structure obtained by spirally winding a thin belt-like plate into a substantially circular tube shape. The band-like plate is formed of a metal material such as stainless steel.

The mesh tube 12 serving as the braid layer is a structure that is obtained by forming a metal mesh member into a substantially circular tube shape, and is so disposed as to cover an outer surface (as to be in contact with an outer periphery) of the spiral tube 11. The metal mesh member is obtained by braiding, into a net shape, strand bundles each including a plurality of strands. The strands are each made of a metal such as stainless steel.

The outer skin 13 serving as the outer resin layer is a structure obtained by forming a flexible resin material such as elastomer into a substantially circular tube shape. The outer skin 13 is so disposed as to cover an outer surface (as to be in contact with an outer periphery) of the mesh tube 12.

Here, the tubular member having the three-layer structure that includes the spiral tube 11, the mesh tube 12, and the outer skin 13 is referred to as a three-layered tube 20. The flexible tube 7 according to the present embodiment mainly includes the three-layered tube 20, an inner pipe sleeve 14 that is an inner cylindrical member, and an outer pipe sleeve 15 that is an outer cylindrical member (see Fig. 5).

The inner pipe sleeve 14 is fitted to an inside of an end part 21 on the proximal end side of the three-layered tube 20. The inner pipe sleeve 14 is provided in order to bundle the end part 21 of the three-layered tube 20. In addition, the inner pipe sleeve 14 and the outer pipe sleeve 15 are connection parts to couple a proximal end portion of the flexible tube 7 to the distal end portion of the operation section 3, as described later.

The inner pipe sleeve 14 is a structure that includes a cylinder portion 14b and an outward flange portion 14a. The cylinder portion 14b is formed of, for example, a metal material in a cylindrical shape as a whole. The outward flange portion 14a is provided at one end part of the cylinder portion 14b.

Here, a form of the three-layered tube 20 before the flexible tube 7 is assembled is as illustrated in Fig. 2. In the state illustrated in Fig. 2, the three-layered tube 20 is in an unloaded state. The state is regarded as a normal state. When the three-layered tube 20 is in the normal state, an inner diameter (see a reference sign D1 in Fig. 2) of the spiral tube 11 is substantially fixed from the distal end to the proximal end. Further, in the normal state, the inner diameter (see a reference sign D1 in Fig. 2) of the end part 21 of the three-layered tube 20 (the spiral tube 11) and an inner diameter (see a reference sign D2 in Fig. 2) of the inner pipe sleeve 14 (the inner cylindrical member) are made substantially equal to each other (D1≈D2).

When the inner pipe sleeve 14 (the inner cylindrical member) is disposed inside the end part 21 of the three-layered tube 20 in the state of Fig. 2, a portion of the flexible tube 7 is assembled in a form illustrated in Fig. 3. To do so, the cylinder portion 14b of the inner pipe sleeve 14 is fitted into the end part 21 of the three-layered tube 20 while the diameter of the end part 21 of the three-layered tube 20 is enlarged to a size larger than an outer periphery of the cylinder portion 14b of the inner pipe sleeve 14 (in other words, force in an arrow B direction of Fig. 2 is applied to the end part 21 to enlarge the diameter). Here, the site at which the diameter of the end part 21 of the three-layered tube 20 is enlarged is denoted by a reference sign 11a in Fig. 3. In the following, the site denoted by the reference sign 11a is referred to as an enlarged-diameter part 11a.

At this time, the end part 21 of the three-layered tube 20 is positioned in an axial direction (an insertion direction) by the outward flange portion 14a of the inner pipe sleeve 14. Application of the enlarging force in the arrow B direction to the end part 21 of the three-layered tube 20 is released after fitting of the inner pipe sleeve 14 to the three-layered tube 20 is completed. This brings the end part 21 of the three-layered tube 20 (the spiral tube 11) into contact with the outer periphery of the cylinder portion 14b of the inner pipe sleeve 14 by biasing force of the spiral tube 11. At this time, the enlarged-diameter state of the end part 21 of the three-layered tube 20 (the spiral tube 11) is maintained by the cylinder portion 14b of the inner pipe sleeve 14. The three-layered tube 20 and the inner pipe sleeve 14 are assembled in such a manner, which causes the portion of the flexible tube 7 according to the present embodiment to be assembled in the form illustrated in Fig. 3.

In the state illustrated in Fig. 3, the inner pipe sleeve 14 (the inner cylindrical member) is disposed inside the end part 21 of the three-layered tube 20 (the spiral tube 11). At this time, the diameter of the end part 21 of the three-layered tube 20 (the spiral tube 11) is enlarged at the enlarged-diameter part 11a. Therefore, the inner pipe sleeve 14 is disposed inside the end part 21 of the three-layered tube 20 (the spiral tube 11) such that the diameter of the end part 21 of the three-layered tube 20 (the spiral tube 11) becomes larger than a diameter of a part 22 that is different from the end part 21 of the three-layered tube 20 (the spiral tube 11).

Note that, as illustrated in Fig. 3, the end part 21 of the three-layered tube 20 (the spiral tube 11) refers to a part at which the inner periphery of the spiral tube 11 is in contact with the outer periphery of the inner pipe sleeve 14 in the state in which the inner pipe sleeve 14 is assembled to the three-layered tube 20 (in the state of Fig. 3). In addition, the part 22 that is different from the end part 21 of the three-layered tube 20 (the spiral tube 11) refers to a part at which the inner periphery of the spiral tube 11 is not in contact with the outer periphery of the inner pipe sleeve 14 in the same state.

Here, a reference sign D1A illustrated in Fig. 3 indicates an inner diameter of the enlarged-diameter part 11a in the end part 21 of the three-layered tube 20 (the spiral tube 11). Further, a reference sign D1 illustrated in Fig. 3 indicates an inner diameter of the part 22 that is different from the end part 21 of the three-layered tube 20 (the spiral tube 11) (in the normal state of Fig. 2, the inner diameter of the end part 21 is substantially equal to the reference sign D1). In other words, the inner diameter D1 of the part different from the end part 21 is smaller than the inner diameter D1A of the enlarged-diameter part 11a (D1<D1A). In addition, a reference sign D2 illustrated in Fig. 3 indicates the inner diameter of the inner pipe sleeve 14.

In the state of Fig. 3, namely, in the state in which the portion (the three-layered tube 20 and the inner pipe sleeve 14) of the flexible tube 7 is assembled, the inner diameter (the reference sign D2 of Fig. 3) of the inner pipe sleeve 14 is substantially equal to (D1≈D2) or at least larger than (D1<D2) the inner diameter (the reference sign D1 of Fig. 3) of the part 22 that is different from the end part 21 of the spiral tube 11 in the three-layered tube 20.

The portion of the flexible tube 7 (the structure in which the three-layered tube 20 and the inner pipe sleeve 14 are assembled) configured in such a manner is connected and fixed to the outer pipe sleeve 15 that is an outer cylindrical member fixed and disposed in the operation section 3.

The outer pipe sleeve 15 is a structure that includes a cylindrical portion 15b and an inward flange portion 15a. The cylindrical portion 15b is formed of, for example, a metal material in a cylindrical shape as a whole. The inward flange portion 15a is provided at one end part of the cylindrical portion 15b. The outer pipe sleeve 15 is disposed outside the end part 21 of the three-layered tube 20. In this case, the end part 21 of the three-layered tube 20 is held between the outer pipe sleeve 15 and the inner pipe sleeve 14. Further, in the state, namely, in the state in which the end part 21 of the three-layered tube 20 is held between the outer pipe sleeve 15 and the inner pipe sleeve 14, the outer pipe sleeve 15 is swaged toward the end part 21 of the three-layered tube 20 (the spiral tube 11). This causes the end part 21 of the three-layered tube 20 to be held between the outer pipe sleeve 15 and the inner pipe sleeve 14 and fixed.

To assemble the end part 21 of the portion of the flexible tube 7 (the structure in which the three-layered tube 20 and the inner pipe sleeve 14 are assembled) to the outer pipe sleeve 15, the structure is first inserted into the outer pipe sleeve 15. At this time, the structure is positioned in the axial direction (the insertion direction) by the inward flange portion 15a of the outer pipe sleeve 15. In the state, swaging force in an arrow F direction illustrated in Fig. 4 is applied to the cylindrical portion 15b of the outer pipe sleeve 15. This causes a portion of the outer pipe sleeve 15 to be swaged, which results in a state illustrated in Fig. 5. As a result, the end part 21 of the three-layered tube 20 is held between the outer pipe sleeve 15 and the inner pipe sleeve 14 and fixed.

Note that the distal end portion, namely, the site coupled to the bending portion 6, of the flexible tube 7 also has a similar configuration. The illustration and description of the distal end portion of the flexible tube 7 are omitted because the portion does not directly relate to the present embodiment.

As mentioned above, according to the above-described first embodiment, the inner pipe sleeve 14 (the inner cylindrical member) of the endoscope flexible tube is disposed inside the end part 21 of the three-layered tube 20 (the spiral tube 11). At this time, the inner pipe sleeve 14 (the inner cylindrical member) is disposed inside the end part 21 of the three-layered tube 20 (the spiral tube 11) such that the diameter of the end part 21 of the three-layered tube 20 (the spiral tube 11) (more specifically, for example, the inner diameter D1 of the spiral tube 11 in the normal state) becomes larger than the diameter of the part 22 that is different from the end part 21 of the three-layered tube 20 (the spiral tube 11). As a result, the inner diameter D2 of the inner pipe sleeve 14 (the inner cylindrical member) is substantially equal to (D1≈D2) or at least larger than (D1<D2) the inner diameter D1 of the part 22 that is different from the end part 21 of the three-layered tube 20 (the spiral tube 11).

Here, the end part 21 of the three-layered tube 20 (the spiral tube 11) is disposed while the diameter of the end part 21 is enlarged to the size larger than the outer periphery of the inner pipe sleeve 14 (the inner cylindrical member). In addition, the end part 21 of the three-layered tube 20 (the spiral tube 11), namely, the end part 21 of the spiral tube 11, the mesh tube 12, and the outer skin 13 is held between the inner pipe sleeve 14 (the inner cylindrical member) and the outer pipe sleeve 15 (the outer cylindrical member) and fixed. Here, the outer pipe sleeve 15 (the outer cylindrical member) is swaged toward the end part 21 of the three-layered tube 20 (the spiral tube 11), which fixes the end part 21.

Such a configuration allows the inner diameter D1 of the spiral tube 11 to be substantially equal to the inner diameter D2 of the inner pipe sleeve 14 (D1≈D2), or allows the inner diameter D2 of the inner pipe sleeve 14 to be at least larger than the inner diameter D1 of the spiral tube 11 (D1<D2). This makes it possible to secure larger internal space of the inner pipe sleeve 14 as compared with the endoscope flexible tube having the conventional structure. Accordingly, it is possible to secure the storage quantity of built-in components in the internal space that is equivalent to or larger than the storage quantity of the conventional configuration.

In addition, since it is possible to make the diameter of the inner pipe sleeve 14 larger than the conventional diameter, a gap between the outer pipe sleeve 15 and the inner pipe sleeve 14 is reduced, which makes it possible to further decrease a swaging amount in swaging of the outer pipe sleeve 15. This contributes to reduction of a work load in the manufacturing process.

### [Second Embodiment]

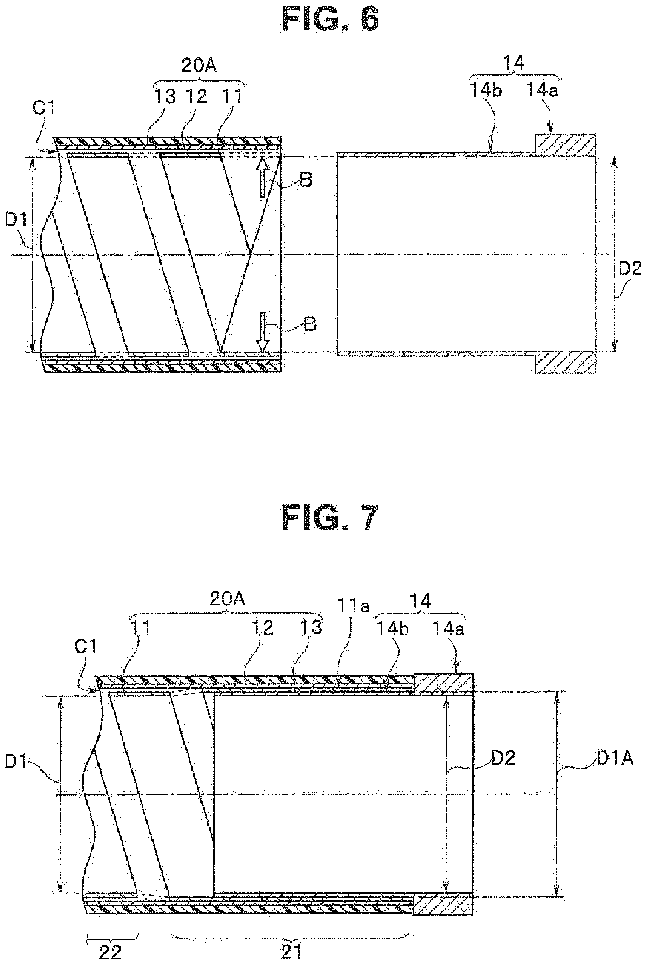

Next, an endoscope flexible tube according to a second embodiment of the present invention is described below with reference to Fig. 6 to Fig. 9.

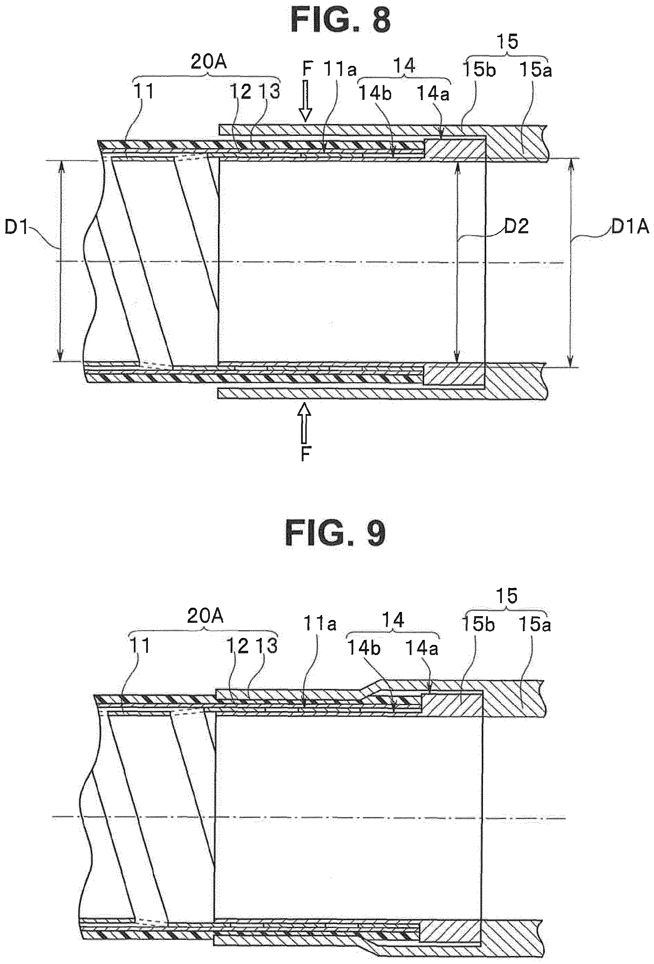

Fig. 6 to Fig. 9 are diagrams each illustrating the second embodiment of the present invention. Among the drawings, Fig. 6 is a diagram illustrating a portion of components on proximal end side of the endoscope flexible tube according to the present embodiment, and is a main-part exploded cross-sectional diagram illustrating a three-layered tube and an inner pipe sleeve in an exploded manner. Fig. 6 corresponds to Fig. 2 in the above-described first embodiment. Fig. 7 is a diagram illustrating a state in which the three-layered tube and the inner pipe sleeve that are in an exploded state in Fig. 6 are assembled. Fig. 7 corresponds to Fig. 3 in the above-described first embodiment. Fig. 8 is a diagram illustrating a state in which an end part of the three-layered tube and an end part of the inner pipe sleeve are inserted into an outer pipe sleeve, in the endoscope flexible tube according to the present embodiment. Fig. 8 corresponds to Fig. 4 in the above-described first embodiment. Fig. 9 is a diagram illustrating a state in which the end part of the three-layered tube and the end part of the inner pipe sleeve have been assembled to the outer pipe sleeve (a state in which swaging work has been completed) in the endoscope flexible tube according to the present embodiment. Fig. 9 corresponds to Fig. 5 in the above-described first embodiment.

A basic configuration according to the present embodiment is substantially similar to the configuration of the first embodiment mentioned above. In the present embodiment, out of the components of the endoscope flexible tube, only the configuration of the three-layered tube that includes the spiral tube, the mesh tube, and the outer skin is slightly different from the configuration in the first embodiment. Accordingly, detailed description of the components same as the components in the above-described first embodiment is omitted, and different portions are only described in detail below.

In the endoscope flexible tube according to the present embodiment, a three-layered tube 20A includes the spiral tube 11, the mesh tube 12, and the outer skin 13, as with the above-described first embodiment. As illustrated in Fig. 3, however, the three-layered tube 20A according to the present embodiment includes a certain clearance (a gap: a reference sign C1 in Fig. 6) between the spiral tube 11 and the mesh tube 12. Providing such a clearance C1 is to smoothly bend the flexible tube without obstacle. In other words, when the clearance C1 is provided between the spiral tube 11 and the mesh tube 12, it is possible to reduce friction between the spiral tube 11 and the mesh tube 12. Since the flexible tube is smoothly bent as the friction is smaller, handling is facilitated and insertion property is improved advantageously.

To assemble the inner pipe sleeve 14 to the end part 21 of the three-layered tube 20A having such a configuration, only the diameter of the spiral tube 11 is enlarged at the end part 21 of the three-layered tube 20A and the inner pipe sleeve 14 is disposed inside the spiral tube 11, as with the above-described first embodiment. This results in a form illustrated in Fig. 7. In the state, as mentioned above, the inner pipe sleeve 14 (the inner cylindrical member) is disposed inside the end part 21 of the spiral tube 11 of the three-layered tube 20A. At this time, the enlarged-diameter state (the inner diameter D1A) of the end part 21 of the three-layered tube 20A (the spiral tube 11) is maintained. This is because the diameter of the inner pipe sleeve 14 is made larger than the inner diameter D1 of the end part 21 of the three-layered tube 20A (the spiral tube 11).

In other words, by the configuration, the inner pipe sleeve 14 is disposed inside the end part 21 of the three-layered tube 20A (the spiral tube 11) such that the diameter of the end part 21 of the three-layered tube 20A (the spiral tube 11) becomes larger than the diameter of the part 22 that is different from the end part 21 of the three-layered tube 20A (the spiral tube 11), also in the present embodiment. As a result, the inner diameter D2 of the inner pipe sleeve 14 (the inner cylindrical member) is substantially equal to (D1≈D2) or at least larger than (D1<D2) the inner diameter D1 of the part 22 different from the end part 21 of the three-layered tube 20A (the spiral tube 11).

The portion of the components of the flexible tube configured in such a manner, namely, the structure in which the three-layered tube 20A and the inner pipe sleeve 14 are assembled is disposed inside the outer pipe sleeve 15 through means similar to the means described in the above-described first embodiment (see Fig. 8), and is swaged. This results in the endoscope flexible tube having a form illustrated in Fig. 9.

As mentioned above, in the present embodiment, when the inner pipe sleeve 14 is assembled to the three-layered tube 20A that includes the spiral tube 11, the mesh tube 12, and the outer skin 13, the outer pipe sleeve 15 is swaged while adjacent members of the inner pipe sleeve 14, the spiral tube 11, the mesh tube 12, the outer skin 13, and the outer pipe sleeve 15 that are provided in order from the inner side are in contact with and overlapped with each other, at the end part 21 of the spiral tube 11. In contrast, the clearance C1 is retained between the outer periphery of the spiral tube 11 and the inner periphery of the mesh tube 12, at the part 22 that is different from the end part 21 of the spiral tube 11.

As mentioned above, the second embodiment makes it possible to provide effects similar to the effects of the above-described first embodiment by configuring, similarly to the configuration of the above-described first embodiment, the three-layered tube 20A that has the large clearance between the spiral tube 11 serving as a flex layer and the mesh tube 12 serving as a braid layer.

Note that the present invention is not limited to the above-described embodiments, and various modifications and applications may be implemented without departing from the scope of the invention as a matter of course. Further, the above-described embodiments include inventions in various stages, and various inventions may be extracted through appropriate combination of the plurality of disclosed components. For example, even if some components are deleted from all the components described in the above-described embodiment, a configuration with the components deleted may be extracted as the invention if such a configuration can solve a problem to be solved by the invention and provide the effects of the invention. Further, components in different embodiments may be appropriately combined.
The present invention is not limited by a specific embodiment, except as limited by the accompanying claims.

The present application is based upon and claims the benefit of priority of the Japanese Patent Application No. 2015-154274 filed in the Japan Patent Office on August 4, 2015.

The contents disclosed by the basic application are incorporated in the present specification, claims, and drawings by reference.

### Industrial Applicability

The present invention is applicable not only to an endoscope in a medical field but also to an endoscope in an industrial field.

## Claims

1. An endoscope flexible tube, comprising:
a spiral tube configured such that a belt-like plate is spirally wound;
an inner cylindrical member disposed inside an end part of the spiral tube; and
an outer cylindrical member that is disposed outside the end part of the spiral tube and holds the end part of the spiral tube between the outer cylindrical member and the inner cylindrical member, wherein
the inner cylindrical member is disposed inside the end part of the spiral tube to make a diameter of the end part of the spiral tube larger than a diameter of a part that is different from the end part of the spiral tube.

2. The endoscope flexible tube according to claim 1, wherein the inner cylindrical member has an inner diameter equal to or at least larger than an inner diameter of the part that is different from the end part of the spiral tube.

3. The endoscope flexible tube according to claim 2, wherein the outer cylindrical member is swaged toward the end part of the spiral tube.

4. The endoscope flexible tube according to claim 3, further comprising:
a mesh tube disposed to be in contact with an outer periphery of the spiral tube; and
an outer skin disposed to be in contact with an outer periphery of the mesh tube, wherein
an end part of the spiral tube, an end part of the mesh tube, and an end part of the outer skin are held between the inner cylindrical member and the outer cylindrical member and fixed.

5. The endoscope flexible tube according to claim 4, wherein the part different from the end part of the spiral tube is disposed to form a clearance between the part and an inner periphery of the mesh tube.

6. The endoscope flexible tube according to claim 4, wherein the end part of the spiral tube, the end part of the mesh tube, and the end part of the outer skin are disposed to be in contact with an outer periphery of the inner cylindrical member while being enlarged in diameter.
